# EUROPEAN PATENT SPECIFICATION

(11) **EP 2 727 542 B1**
(45) Date of publication and mention of the grant of the patent: **10.06.2015**
(21) Application number: 12190941.0
(22) Date of filing: 01.11.2012
(51) Int. Cl.: A61B 17/17

(54) **Lateral light-emitting device for connecting an intra-medullary guide wire**
Seitliche lichtemittierende Vorrichtung zum Verbinden eines intramedullären Führungsdrahtes
Dispositif électroluminescent latéral pour connecter un fil de guidage intramédullaire

(43) Date of publication of application: 07.05.2014
(73) Proprietor: National Yang-Ming University, Taipei City 112 (TW)
(72) Inventor: Chu, Woei-Chyn, 112 Taipei (TW); Tseng, Yin-Jiun, 112 Taipei (TW); Chu, William, 112 Taipei (TW)
(74) Representative: Viering, Jentschura & Partner Patent- und Rechtsanwälte

(56) References cited:
- WO-A2-2007/131231
- DE-A1- 4 344 470

## Description

### BACKGROUND

### Field

This disclosure relates generally to a lateral light-emitting device capable of connecting an intra-medullary guide wire.

### Description of Related Art

Lower extremities fractures are treated by intra-medullary nailing that is a golden standard method for the recent twenty years. The insertion of the nail requires a guide wire to guide the insertion of the intra-medullary nail into the medullary cavity.

Interlocking nail provides good fixation thus better treatment efficacy over the conventional medullary nails. During operation, it must be localized the distal and proximal screw holes and then use screws to fix the nail (locking the nail). Because the nail is embedded in the medullary cavity, so a standard approach is to use the roentgenography to "see" the screw holes and completed this procedure.

Please reference to U.S. issued patent No. 5,417,688. The optical distal targeting system and method for an intra-medullary nail for detecting the location of transverse holes of an intra-medullary nail which has been inserted into a long bone and for aligning a surgical drill to the transverse holes is disclosed. An insert having a light source at its distal end emitting non-ionizing electromagnetic radiation in the visible spectrum or infrared is inserted into the intra-medullary nail, such that the light source is placed adjacent to the transverse holes. The light source may be a directional radiation output emitting the radiation in a direction perpendicular to the axis of the intra-medullary nail. The light source may alternatively be an isotropic radiation output emitting radiation in all directions. The surgeon detects the transmitted radiation on the surface of the body of the patient and aligns the drill with the emitted radiation.

Please also reference to TW issued patent No. 485036. A medullary guide wire with a self-attached lighting device is disclosed. The front end of the guide wire is self-attached with a lighting device, and the visible light or near infrared light from the lighting device will penetrate out of the tissues and bone surface and is used for detecting the screw hole. During screw hole detection, the light-emitting medullary guide wire is stayed in the nail inserted into the marrow cavity and the light emitted through the screw hole and the bone surface is observed from outside of the body. The light-emitting guide wire enables a quick and convenient way to position the screw holes while performing the intra-medullary nailing procedure.

The two structures of the above mentioned prior arts are used by illuminating from within the body to proceed the operation so as to reduce the risk of additional traumas and improve the efficacy of operations, but because the light source is arranged at the front end of the guide wire so a significant portion of the emitted light will travel axially and is not useful for detecting the screw hole which lies on the lateral side of the nail. It may thus require to increase the light power that caused higher power consumption or even injuries resulting from high temperature generated from the high light intensity.

The structure disclosed in a U.S. issued patent No. 5,417,688 has a hole dug laterally so as to divert the light to emit through lateral sides. However, because the light source is disposed in an open structure, the lighting device is easily permeated by the body fluids to cause the device malfunction or contaminate the surrounding tissues.

In addition, the lighting device and the medullary guide wire are fixed in one piece, this increases the manufacturing difficulty as the diameter of the guide wire is only about 3 mm. Furthermore, because the lighting device is arranged at the front end of the medullary guide wire, the medullary guide wire needs to be inserted into the marrow cavity, and the light source has no protecting device to protect it while proceeding the operation, it is easily to damage the light source in the marrow cavity resulting from colliding bone tissue. When this happened, the assembly of the lighting device and the medullary guide wire must be discarded all together. It will be a waste of resources.

Document DE4344470 A1 discloses a lateral light-emitting device for locating distal locking holes of an intramedullary device.

### SUMMARY

The light emitted from the lighting device inserted into the medullary cavity for positioning the screw hole is an effective method in intra-medullary nailing procedures. In order to solve the above mentioned problems and improve the luminous efficiency of the lighting device, a lateral-emitting lighting device capable of working with any kind of intra-medullary guide wire is disclosed.

To achieve the above objective, a lateral-emitting device for connecting a intra-medullary guide wire is disclosed. The device has a stand-alone closed space so that the liquid (e.g. body fluid) is unable to permeate into the device.

Another objective of this invention is to provide a protecting mechanism to the light emitting device. A protecting unit is disposed at the front end of the device for protecting the main body from colliding with the bone tissues resulting in damaging the inner structure and function of the main body.

One another objective of this invention is to provide a lateral-emitting device for connecting an intra-medullary guide wire. The individual main body with a connecting unit is capable of making the lateral-emitting device tightly connected to any kind of intra-medullary guide wire.

One another objective of this invention is to provide a lateral-emitting device for connecting an intra-medullary guide wire. A light-scattering unit is disposed inside the main body so that the light is scattered, brightness enhanced, and laterally emitted to a closed window when the emitted light hit and reflected from the curved surface of the light-scattering unit.

One another objective of this invention is to provide a lateral-emitting device for connecting an intra-medullary guide wire. A light intensity modulating unit is arranged inside the main body to modulate the light intensity to reduce the smear of the scattered light effect.

One another objective of this invention is to provide a lateral-emitting device for connecting an intra-medullary guide wire with a closed main body for preventing the body fluid from permeating in the body to cause contamination and damages. Furthermore, it makes the modulated light passed through the light intensity modulating unit and then laterally emitted through the light-scattering unit to assist fixation of the screw hole.

The lateral light-emitting device for connecting an intra-medullary guide wire, comprising: a main body, having a closed accommodating space thereinside; a front-end protecting unit, disposed in a front end of the main body; a light-scattering unit, disposed in the accommodating space and arranged in back of the front-end protecting unit; a lighting unit, disposed at a predetermined position in the accommodating space of the main body and corresponding to the light-scattering unit so as to make a light generated by the lighting unit be capable of projecting to the light-scattering unit, passing therethrough, and generating scattering; a power supply unit, arranged at the one end of the accommodating space of the main body to provide power to make the lighting unit illuminate; at least one transparent window, disposed at the side of the main body so as to make the scattered light in the accommodating space emit outwardly through the transparent window; and a connecting unit, disposed at the distal end of the main body for connecting the lateral light-emitting device and the medullary guide wire.

### BRIEF DESCRIPTION OF THE DRAWINGS

Various embodiments are depicted in the accompanying drawings for illustrative purposes, and should in no way be interpreted as limiting the scope of the inventions. In addition, various features of different disclosed embodiments can be combined to form additional embodiments, which are part of this disclosure. Any feature or structure can be removed or omitted. Throughout the drawings, reference numbers may be reused to indicate correspondence between reference elements.
FIG. 1 is a structural drawing of a lateral light-emitting device for connecting an intra-medullary guide wire in accordance with the invention.
FIG. 2 is showing a preferable embodiment of the lateral light-emitting device for connecting an intra-medullary guide wire in accordance with the invention.
FIG. 3 is a showing the fixed type intra-medullary nailing used in the tibial fractures by the lateral light-emitting device for connecting an intra-medullary guide wire in accordance with the invention.

### DEDISTALED DESCRIPTION OF PREFERRED EMBODIMENTS

Although certain preferred embodiments and examples are disclosed herein, inventive subject matter extends beyond the examples in the specifically disclosed embodiments to other alternative embodiments and/or uses, and to modifications and equivalents thereof. Thus, the scope of the claims appended hereto is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

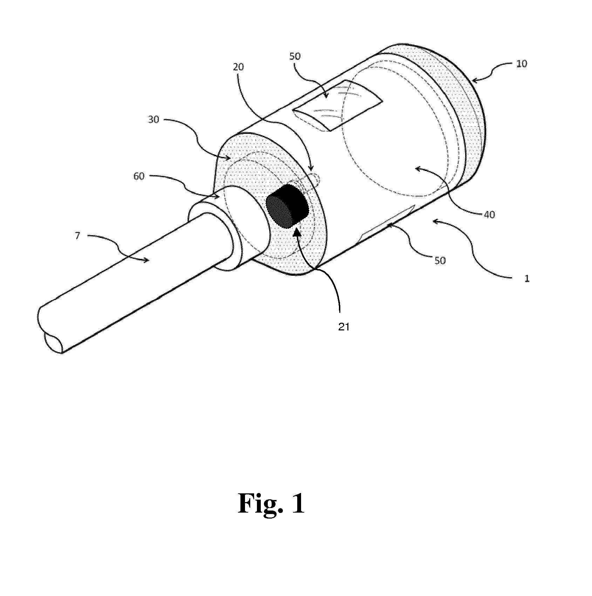

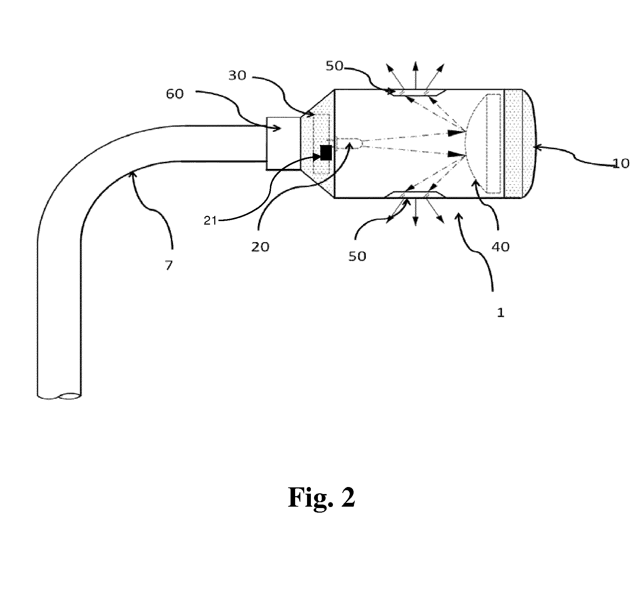

Please refer to FIGs. 1 and 2, which FIG. 1 is a structural drawing of a lateral light-emitting device for connecting an intra-medullary guide wire in accordance with the invention, and FIG. 2 is showing a preferable embodiment of the lateral light-emitting device for connecting an intra-medullary guide wire in accordance with the invention.

Please reference to FIG. 1, the lateral light-emitting device for connecting an intra-medullary guide wire comprises a main body 1. The main body 1 is designed as a close accommodating space to prevent the liquid (e.g. body fluid) from permeating into the body. The outside of the main body 1 is made of transparent, thermal isolation, and bio-compatibility material.

A front-end protecting unit 10 is arranged at the front end of the main body 1 used for preventing the main body 1 from colliding the bone tissues to cause damages while operating treatments. In one embodiment of this invention, the front-end protecting unit 10 is made of a hard material to protect the main body 1 to be capable of being pushed ahead smoothly. Furthermore, the front-end protecting unit 10 may be a hard material with elastic protection function. The front-end protecting unit 10 may be connected the front end of the main body 1 with any kinds of ways, or the front-end protecting unit 10 and the main body 1 are one piece.

A connecting unit 60 is arranged at the distal end of the main body 1 for connecting the intra-medullary guide wire 7. In one embodiment of this invention, the connecting unit 60 is made of an elastic and invaginated material to tightly connecting the main body 1 and the intra-medullary guide wire 7 and released them by outwardly forcing the intra-medullary guide wire 7. In addition, the connecting unit 60 has an outer metal ring (not shown) having set threads or at least one screw for fixing to tighten the outer metal ring (not shown) while fixing the connecting unit 60 and connecting the medullary guide wire 7.

The main body 1 has a light-scattering unit 40 arranged inside the close accommodating space of the main body 1 and including a reflecting surface with a convex arc shape. In one embodiment of this invention, the surface of the light-scattering unit 40 is a glass, plastic sheet, or acrylic sheet plating a metal oxide film capable of reflecting light. In another embodiment of this invention, the reflecting surface of the light-scattering unit 40 has an inclined angle capable of making the straight light be scattered to the transparent window.

A lighting unit 20 is arranged inside the close accommodating space of the main body 1 and corresponding to a predetermined position of the light-scattering unit 40. A power supply unit 30 is arranged at one end of the close accommodating space of the main body 1. The power supply unit 30 may be a micro cell power supply system to provide power to the lighting unit 20. A light intensity modulating unit 21 may be arranged between the lighting init 20 and the power supply unit 30. The light intensity modulating unit 21 has a variable resistor to adjust the magnitude of current flowed into the lighting unit 20. In one embodiment of this invention, a straight light with suitable light intensity is emitted and forward to the light-scattering unit 40 after the power supply unit 30 is powered on to provide power to the lighting unit 20 and then the light intensity of the lighting unit 20 is modulated by the light intensity modulating unit 21.

At least one transparent window 50 is selected from one of following materials for easily making the light pass through: glass, acrylic, and plastic to become a sealed transparent block and arranged at side of the main body 1. The light is emitted outside through the transparent window 50 while the light emitted from the lighting unit 20 arranged in the close accommodating space is scattered by the light-scattering unit 40.

In addition, the surface of the transparent window 50 may be arranged a reflect mirror with prism angles. Accordingly, the light is emitted outwardly after being enhanced and focused.

Please reference to FIG. 2, which is showing the laterally emitting process of the lateral-emitting device for connecting the intra-medullary guide wire. In one embodiment of this invention, the light source of the lighting unit 20 is LED and the power supply unit 30 is battery used for providing power to the lighting device 20. After light intensity of the lighting unit 20 is modulated by the light intensity modulating unit 21, the light is straightly emitted. When the straight light is contacting the light-scattering unit 40, it may be scattered by the arc convex reflecting surface (mirror) of the light-scattering unit 40. The scattered light is emitted to the transparent window 50 with prism angels and modulated and focused through the transparent window 50 to make the focused light emitted from the transparent window 50 laterally.

For the above mentioned, the lateral-emitting device of this invention may change the light emitting direction, and control the laterally emitted light to be focused and emitted the clear light to be easily positioned and observed out of the body.

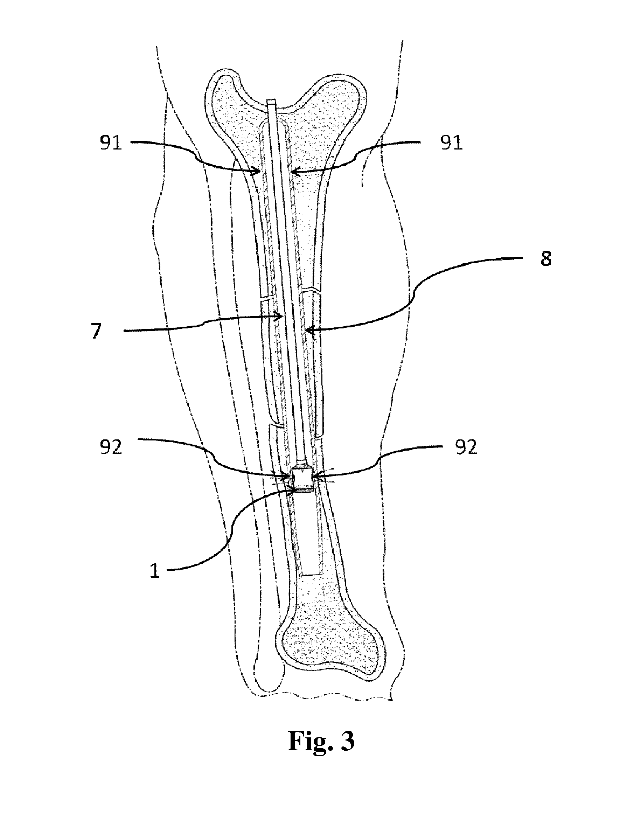

Please reference to FIG. 3, which is showing the fixed type intra-medullary nailing used in the tibial fractures by the lateral light-emitting device for connecting an intra-medullary guide wire in accordance with the invention. The detail steps are:
(1). The lateral-emitting device is connected to one end of a flexible intra-medullary guide wire 7 by the connecting unit 60. The connecting unit 60 has a socket head so as to connect the medullary guide wire 7 tightly by toothed structure with a frictional force therebetween.
(2). When positioning the hole of the nail, it is started from the farther two distal ends 91. The intra-medullary guide wire 7 is retracted backwardly to make the lateral-emitting device 10 be positioned at the distal end 91 of the nail (evaluated by the length of the nail). The power supply unit 30 is to provide the power to the lighting unit 20 of the lateral-emitting device 10. After the light intensity from the lighting unit 20 is modulated by the light intensity modulating unit 21, the lighting unit 20 is emitted the straight light with suitable intensity. The light is scattered after the emitted straight light hits the light-scattering unit 40. The scattered light is laterally emitted through the transparent window 50. Next, the intra-medullary guide wire 7 connected to the lateral-emitting device 10 is moved back and forth till the light goes through from the desired positioning hole. Continue the movement based on the above steps and simultaneously observe the light coming out of the screw hole. If the light intensity is gradually enhanced, continue moving in the same direction. If not, move in the opposite direction until the rough location of the desirable positioned hole is determined. The light source of the lighting unit 20 is moved back and forth gradually near the determined rough location till the location of the brightest point is capable of being determined. And further change the observing angle till a symmetrical complete circle bright point is observed. The center of the circle is a center of the screw hole of the nail. Mark the location of the center point.
(3). The intra-medullary guide wire 7 is retracted backwardly later to make the light source of the lateral-emitting device 10 move away from the marked center at previous step. The skin with the marked center is incised own to the bone, the bone is drilled by a drill, and then the fixed screw is locked therein.
(4). Repeat steps (2) and (3) to the rest distal screw holes 91 and proximal screw holes 92, and lock into the fixed screw.

After above mentioned steps, the lighting effect is good to make the holes of the nail clear while the intra-medullary connecting the lateral-emitting device is used for fixed type intra-medullary nailing. The light source may not be damaged or broken during the process and the body fluid may not be permeated after operating with the lateral-emitting device.

It should be appreciated that in the above description of embodiments, various features are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular embodiment herein can be applied to or used with any other embodiment(s). Thus, it is intended that the scope of the inventions herein disclosed should not be limited by the particular embodiments described above, but should be determined only by a fair reading of the claims that follow.

## Claims

1. A lateral light-emitting device for connecting an intra-medullary guide wire, (7) comprising:
a main body (1), , having a close accommodating space thereinside;
a front-end protecting unit(10), disposed in a front end of the main body;
a light-scattering unit (40), disposed in the accommodating space and arranged in back of the front-end protecting unit;
a lighting unit (20), disposed at a predetermined position in the accommodating space of the main body and corresponding to the light-scattering unit so as to make a light generated by the lighting unit be capable of projecting to the light-scattering unit, passing therethrough, and generating scattering;
a power supply unit (30), arranged at one end of the accommodating space of the main body to provide power to make the lighting unit illuminate;
at least one transparent window (50), disposed at side of the main body so as to make the scattered light in the accommodating space emit outwardly through the transparent window; and
a connecting unit (60), disposed at a distal end of the main body for connecting the lateral light-emitting device and the medullary guide wire.

2. The device of claim 1, wherein the main body is made of transparent, thermal isolation, and bio-compatibility material.

3. The device of claim 1, wherein the front-end protecting unit is made of a hard material to protect the main body to be capable of being pushed ahead smoothly.

4. The device of claim 1, wherein a surface of the transparent window has a reverse cone shape with a light-reflecting surface so as to make the light emit outwardly easily.

5. The device of claim 1, wherein the transparent window is selected from one of following materials for easily making the light pass through: glass, acrylic, and plastic.

6. The device of claim 1, wherein a surface of the light-scattering unit is a reflecting surface with a convex arc shape.

7. The device of claim 6, wherein the surface of the light-scattering unit is a glass, plastic sheet, or acrylic sheet plating a metal oxide film.

8. The device of claim 6, wherein the reflecting surface of the light-scattering unit has an inclined angle capable of making the straight light be scattered to the transparent window.

9. The device of claim 1, wherein the power supply unit is arranged a micro cell thereinside to provide power to the lighting unit.

10. The device of claim 1, further comprises a light intensity modulating unit arranged between the lighting unit and the power supply unit so as to modulate light intensity of the lighting unit.

11. The device of claim 10, wherein the light intensity modulating unit has a variable resistor to adjust the magnitude of current flowed into the lighting unit.

12. The device of claim 1, wherein the connecting unit has a socket head so as to connect the medullary guide wire tightly by a frictional force therebetween.

13. The device of claim 1, wherein the connecting unit has an outer metal ring having threads or at least one screw for fixing to tighten the outer metal ring while fixing the connecting unit and connecting the medullary guide wire.

## Patentansprüche

1. Eine laterale lichtemittierende Vorrichtung zum Verbinden eines intramedullären Führungsdrahtes (7) umfassend:
einen Hauptkörper (1), der darin innenseitig einen geschlossenen Unterbringungsraum aufweist;
eine stirnseitige Schutzeinheit (10), die an einem vorderen Ende des Hauptkörpers angeordnet ist;
eine Lichtstreuungseinheit (40), die in dem Unterbringungsraum angeordnet ist und an der Rückseite der stirnseitigen Schutzeinheit vorgesehen ist;
eine Lichteinheit (20), die an einer vorbestimmten Position in dem Unterbringungsraum des Hauptkörpers und entsprechend zu der Lichtstreuungseinheit angeordnet ist, damit das durch die Lichteinheit erzeugte Licht auf die Lichtstreuungseinheit projizieren kann, dadurch passieren kann und eine Streuung erzeugt;
eine Stromversorgungseinheit (30), die an einem Ende des Unterbringungsraums des Hauptkörpers vorgesehen ist, um Energie bereitzustellen, um die Lichteinheit zu erleuchten:
zumindest ein transparentes Fenster (50), das an einer Seite des Hauptkörpers angeordnet ist, damit das gestreute Licht in dem Unterbringungsraum nach außen durch das transparente Fenster emittieren kann; und
eine Verbindungseinheit (60), die an einem distalen Ende des Hauptkörpers zum Verbinden der lateralen lichtemittierenden Vorrichtung und des medullären Führungsdrahtes angeordnet ist.

2. Die Vorrichtung nach Anspruch 1, wobei der Hauptkörper aus einem transparenten, thermisch isolierenden und biokompatiblen Material besteht.

3. Die Vorrichtung nach Anspruch 2, wobei die stirnseitige Schutzeinheit aus einem harten Material besteht, um den Hauptkörper zu schützen, damit er sanft nach vorne gedrückt werden kann.

4. Die Vorrichtung nach Anspruch 1, wobei eine Oberfläche des transparenten Fensters eine umgekehrte konische Form hat mit einer lichtreflektierenden Oberfläche, damit das Licht leicht nach außen emittieren kann.

5. Die Vorrichtung nach Anspruch 1, wobei das transparente Fenster aus einem der folgenden Materialien ausgewählt ist, damit das Licht leicht dadurch passieren kann: Glas, Acryl und Kunststoff.

6. Die Vorrichtung nach Anspruch 1, wobei eine Oberfläche der Lichtstreuungseinheit eine reflektierende Oberfläche mit einer konvexen Bogenform hat.

7. Die Vorrichtung nach Anspruch 6, wobei die Oberfläche der Lichtstreuungseinheit ein Glas, eine Kunststofffolie oder eine einen Metalloxidfilm beschichtende Acrylfolie ist.

8. Die Vorrichtung nach Anspruch 6, wobei die reflektierende Oberfläche der Lichtstreuungseinheit einen geneigten Winkel aufweist, damit das geradeaus gerichtete Licht zu dem transparenten Fenster gestreut werden kann.

9. Die Vorrichtung nach Anspruch 1, wobei die Stromversorgungseinheit als eine Mikrozelle darin innenseitig vorgesehen ist, um die Lichteinheit mit Energie zu versorgen.

10. Die Vorrichtung nach Anspruch 1, ferner umfassend eine Lichtintensitätsmodulierungseinheit, die zwischen der Lichteinheit und der Stromversorgungseinheit vorgesehen ist, um so die Lichtintensität der Lichteinheit zu modulieren.

11. Die Vorrichtung nach Anspruch 10, wobei die Lichtintensitätsmodulierungseinheit einen variablen Widerstand aufweist, um die Stärke des in die Lichteinheit fließenden Stromes anzupassen.

12. Die Vorrichtung nach Anspruch 1, wobei die Verbindungseinheit einen Steckkopf aufweist, um so den medullären Führungsdraht fest mittels einer Friktionskraft dazwischen zu verbinden.

13. Die Vorrichtung nach Anspruch 1, wobei die Verbindungseinheit einen äußeren Metallring mit Einschraubgewinden oder zumindest einer Schraube zum Befestigen aufweist, um den äußeren Metallring festzuziehen während des Befestigens der Verbindungseinheit und des Verbindens des medullären Führungsdrahtes.

## Revendications

1. Dispositif d'émission d'une lumière latérale, destiné à connecter un fil guide intramédullaire (7), comprenant :
un corps principal (1), qui présente à l'intérieur un espace de réception étroit ;
une unité de protection de l'extrémité avant (10), disposée dans l'extrémité avant du corps principal ;
une unité de diffusion de la lumière (40), disposée dans l'espace de réception et agencée à l'arrière de l'unité de protection de l'extrémité avant ;
une unité d'éclairage (20), disposée au niveau d'une position prédéterminée dans l'espace de réception du corps principal et qui correspond à l'unité de diffusion de la lumière de façon à ce qu'une lumière générée par l'unité d'éclairage puisse être projetée vers l'unité de diffusion de la lumière, en passant à travers, et en générant une diffusion ;
une unité d'alimentation électrique (30), agencée au niveau d'une extrémité de l'espace de réception du corps principal, destinée à fournir de l'énergie de telle sorte que l'unité d'éclairage éclaire ;
au moins une fenêtre transparente (50), disposée au niveau d'un côté du corps principal de façon à ce que la lumière diffusée dans l'espace de réception soit émise vers l'extérieur à travers la fenêtre transparente ; et
une unité de connexion (60), disposée au niveau d'une extrémité distale du corps principal, destinée à connecter le dispositif d'émission d'une lumière latérale et le fil guide médullaire.

2. Dispositif selon la revendication 1, dans lequel le corps principal est réalisé dans un matériau transparent, calorifuge et biocompatible.

3. Dispositif selon la revendication 1, dans lequel l'unité de protection de l'extrémité avant est réalisée dans un matériau dur de façon à protéger le corps principal de manière à pouvoir le pousser en avant et sans à-coup.

4. Dispositif selon la revendication 1, dans lequel une surface de la fenêtre transparente présente une forme de cône renversé avec une surface réfléchissante de façon à ce que la lumière soit facilement émise vers l'extérieur.

5. Dispositif selon la revendication 1, dans lequel la fenêtre transparente est sélectionnée à partir de l'un des matériaux suivants de façon à faciliter le passage de la lumière à travers celle-ci : verre, acrylique et matière plastique.

6. Dispositif selon la revendication 1, dans lequel une surface de l'unité de diffusion de la lumière est une surface réfléchissante qui présente une forme d'arc convexe.

7. Dispositif selon la revendication 6, dans lequel la surface de l'unité de diffusion de la lumière est un verre, une feuille de matière plastique ou une feuille acrylique recouvrant une pellicule d'oxyde métallique.

8. Dispositif selon la revendication 6, dans lequel la surface réfléchissante de l'unité de diffusion de la lumière présente un angle incliné qui permet de diffuser la lumière droit vers la fenêtre transparente.

9. Dispositif selon la revendication 1, dans lequel est disposée à l'intérieur de l'unité d'alimentation électrique une micropile de façon à fournir de l'énergie à l'unité d'éclairage.

10. Dispositif selon la revendication 1, comprenant en outre une unité de modulation de l'intensité de la lumière agencée entre l'unité d'éclairage et l'unité d'alimentation électrique, destinée à moduler l'intensité de la lumière de l'unité d'éclairage.

11. Dispositif selon la revendication 10, dans lequel l'unité de modulation de l'intensité de la lumière présente un résisteur variable destiné à régler la grandeur du courant qui circule dans l'unité d'éclairage.

12. Dispositif selon la revendication 1, dans lequel l'unité de connexion présente une tête creuse de façon à connecter le fil guide médullaire étroitement à celle-ci grâce à une force de friction.

13. Dispositif selon la revendication 1, dans lequel l'unité de connexion présente un anneau métallique extérieur qui présente un filetage ou au moins une vis de fixation, destiné à serrer l'anneau métallique extérieur tout en fixant l'unité de connexion et en connectant le fil guide médullaire.
